# EUROPEAN PATENT APPLICATION

(11) **EP 1 369 038 A1**
(43) Date of publication of application: **10.12.2003**
(21) Application number: 02405451.2
(22) Date of filing: 05.06.2002
(51) Int. Cl.: A01N 31/16, A01N 31/14, A61K 7/48, A61K 7/06

(54) **Personal care products**

(71) Applicant: Ciba Specialty Chemicals Holding Inc., 4057 Basel (CH)
(72) Inventor: Stehlin, Albert., 68128 Rosenau (FR)

(57) **Abstract**

The present invention provides a personal care product, in particar a soap, with antimicrobial action containing, as antimicrobial compound, a compound formula 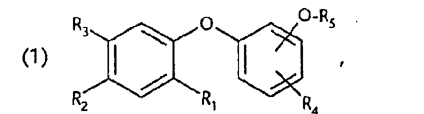 wherein the substituents are as defined in the claims; and a UV-absorber
which has improved properties by diminishing the discoloration of the personal care products caused by the antimicrobial agent.

## Description

The present invention relates to personal care products with antimicrobial action, which has improved properties against the diminishing of the discoloration of the personal care products caused by the antimicrobial agent.

Hydroxydiphenylethers are known antimicrobial compounds, which can also be incorporated in soaps in order to impart to these e.g. a disinfecting action. Many of these compounds, e.g. halogenated hydroxydiphenyl ethers, which are excellent antimicrobial compounds and therefore also extremely effective in soaps (cf. for example British patent specification No. 1,024,022), have the disadvantage that they cause yellowing in bars of soap when these are exposed to light. The bars of soap thus assume an undesired unattractive appearance.

Methods of diminishing and/or avoiding this disadvantage have already been proposed. For example, U.S. Pat. No. 3,284,362 teaches the incorporation of aromatic carboxylic acids as light stabilizers in soaps, whereby a certain improvement in quality of the soap is obtained.

British patent specification No.1,175,408 postulates the incorporation of free straight-chain fatty acids in soaps containing halogenated o-hydroxydiphenyl ethers. A certain improvement in quality is thereby obtained, without at the same time providing a complete solution to the problem. Many of the fatty acids cited are often a regular constituent of finished bars of soap in so-called "superfatted soaps".

It is known from U.S. Pat. No. 4,115,294 that the addition of N-acylsarcosine derivatives reduces the light sensitivity of soaps containing halogenated o-hydroxydiphenyl ethers and thus keeps the deterioration in aspect on exposure to light within bounds.

Finally it is known from US 4,326,978 that the addition of water-insoluble colourless silicate reduces the light sensitivity.

The present invention provides a personal care product with antimicrobial action comprisingng,
(a) as antimicrobial a compound of formula 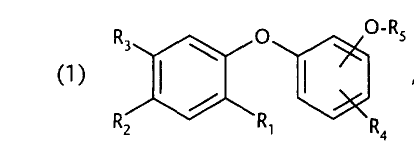 wherein
   - R₁, R₂ and R₃: independently from each other are hydrogen; hydroxy; halogen; C₁-C₂₀alkyl; hydroxy-substituted C₁C₂₀alkyl; C₅-C₇cycloalkyl; C₁-C₂₀alkoxy; C₁-C₆alkylcarbonyl; phenyl; or phenyl-C₁-C₃alkyl;
   - R₄: is hydrogen; halogen; C₁-C₂₀alkyl; hydroxy-substituted C₁-C₂₀alkyl; C₅-C₇cycloalkyl; hydroxy; formyl; acetonyl; allyl; carboxy; carboxy-C₁-C₃alkyl; carboxyallyl; C₂-C₂₀alkenyl; C₁-C₆-alkylcarbonyl; C₁-C₃alkylcarbonyl-C₁-C₃alkyl; phenyl; or phenyl-C₁-C₃alkyl; and
   - R₅: is hydrogen; C₁-C₂₀alkoxy; or C₁-C₆alkylcarbonyl; and
(b) a UV absorber selected from
(b₁) a compound of formula 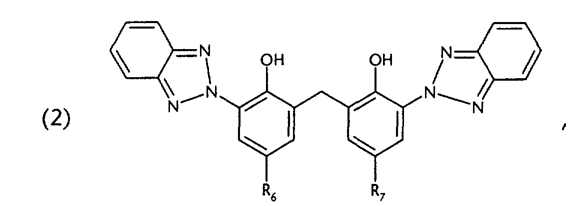 wherein
   - R₆ and R₇: independently from each other signify C₁-C₁₂-alkyl;
(b₂) a compound of formula 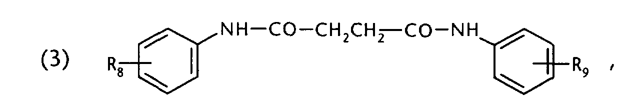 in which R₈ and R₉, independently, are C₁-C₁₈alkyl or C₁-C₁₈alkoxy;
(b₃) a compound of formula 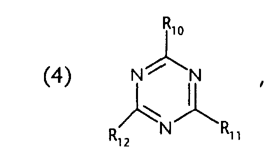 in which
   - R₁₀, R₁₁ and R₁₂,: independently, are H; OH; C₁-C₁₈alkoxy; NH₂; NH-R₁₃ or N(R₁₃)₂in which R₁₃ is C₁-C₁₈alkyl; OR₁₃; phenyl, phenoxy or anilino, or pyrrolo, in which the respective phenyl, phenoxy or anilino, or pyrrolo moieties are optionally substituted by one, two or three substitutents selected from OH, carboxy, CO-NH₂, C₁-C₁₈alkyl or C₁-C₁₈alkoxy; C₁-C₁₈carboxyalkyl; C₅-C₈cycloalkyl; a methylidenecamphor group; a group -(CH=CH)ₘC(=O)-OR₁₃ in which m is 0 or 1; or a group 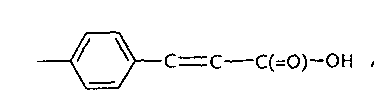 or the corresponding alkali metal, ammonium, mono-, di- or tri-C₁-C₄alkylammonium, mono-, di- or tri-C₂-C₄alkanolammonium salts, or the C₁-C₁₈alkyl esters thereof;
(b₄) a compound of formula 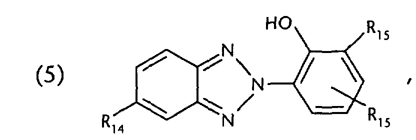 in which
   - R₁₄: is C₁-C₁₈alkyl or hydrogen; and
   - R₁₅: is C₁-C₁₈alkyl, optionally substituted by phenyl,
(b₅) a compound of formula 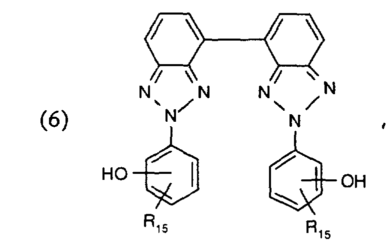 in which R₁₅ has its previous significance;
(b₆) a compound of formula

   (7) R₁₂-(Y)ₘ-CO-C(R₁₇)=C(R₁₈)-N(R₁₉)(R₂₀),

   in which
   - R₁₆: is C₁-C₁₈alkyl or phenyl optionally substituted by one, two or three substituents selected from OH, C₁-C₁₈alkyl, C₁-C₁₈alkoxy or CO-OR₁₃;
   - R₁₇, R₁₈, R₁₉ and R₂₀: are the same or different and each is C₁-C₁₈alkyl; or hydrogen;
   - Y: is N; or O; and
   - m: has its previous significance;
(b₇) a compound of formula 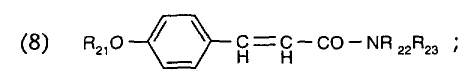 in which
   - R₂₁: is hydroxy; or C₁-C₄alkoxy,
   - R₂₂: is hydrogen; or C₁-C₄alkyl; and
   - R₂₃: is -(CONH)ₘ-phenyl in which m has its previous significance and the phenyl group is optionally substituted by one, two or three substituents selected from OH, C₁-C₁₈alkyl,. C₁-C₁₈alkoxy or CO-OR₁₃; and
(b₈) a compound of formula 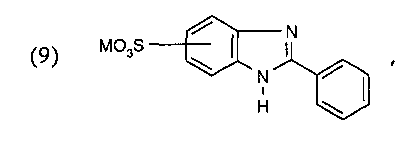 , in which
   - M: is hydrogen; an alkali metal; an alkaline earth metal; or zinc.

Each alkyl radical is straight-chained or branched. Examples of alkyl radicals are methyl, ethyl, n-propyl, isopropyl, n-butyl, sec-butyl, tert-butyl, amyl, isoamyl or tert-amyl, heptyl, octyl, isooctyl, nonyl, decyl, undecyl, dodecyl, tetradecyl, pentadecyl, hexadecyl, heptadecyl, octadecyl or eicosyl.

Examples of alkenyl radicals are allyl, methallyl, isopropenyl, 2-butenyl, 3-butenyl, isobutenyl, n-penta-2,4-dienyl, 3-methyl-but-2-enyl, n-oct-2-enyl, n-dodec-2-enyl, isododecenyl, n-dodec-2-enyl or n-octadec-4-enyl.

Each alkoxy radical is straight-chained or branched. Examples of alkoxy radicals are methoxy, ethoxy, n-propoxy, isopropoxy, n-butoxy, sec-butoxy, tert-butoxy, pentyloxy, iso-pentyloxy, tert-pentyloxy, heptyloxy, octyloxy, isooctyloxy, nonyloxy or decyloxy and the like.

Each alkylcarbonyl is straight-chained or branched. Examples of carbonyl radicals are acetyl, propionyl, butyryl, isobutyryl, valeryl, isovaleryl or pivaloyl and the like.

Examples of hydroxy-substituted C₁-C₂₀alkyl are hydroxymethyl, hydroxyethyl, hydroxypropyl, hydroxybutyl, hydroxypentyl, hydroxyhexyl, hydroxyheptyl, hydroxyoctyl, hydroxynonyl or hydroxydecyl and the like.

C₅-C₇cycloalkyl is cyclopentyl, cycloheptyl or, preferably, cyclohexyl.

C₇-C₉phenylalkyl is phenylpropyl, phenylethyl and, preferably, benzyl.

In a preferred embodiment of the invention the personal care product contains an antimicrobial of formula (1), wherein
- R₁: is C₁-C₁₆-Alkyl; and
- R₂, R₃ R₄ and R₅: are hydrogen.

In a further preferred embodiment of the invention the personal care product contains as antimicrobial a compound of formula 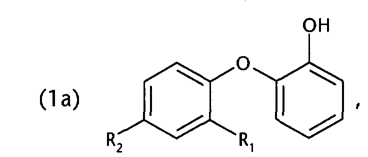 wherein
- R₁ and R₂: independently of each other are hydrogen; C₁-C₂₀alkyl; or hydroxy-substituted C₁-C₂₀alkyl; wherein at least one of the substituents R₁ and R₂ is not hydrogen.

In a more preferred embodiment of the invention the personal care product contains an antimicrobial of formula 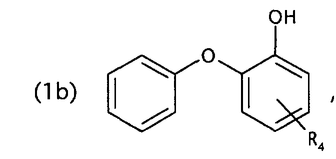 wherein
- R₄: is carboxy; carboxy-C₁-C₃alkyl; C₁-C₆alkylcarbonyl; or C₁-C₃alkylcarbonyl-C₁-C₃alkyl.

In a further preferred embodiment of the present invention the personal care product contains an antimicrobial of formula 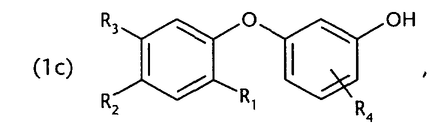 wherein
- R₁, R₂ and R₃: independently from each other are hydrogen; hydroxy; C₁-C₂₀alkyl; or hydroxy-substituted C₁C₂₀alkyl; and
- R₄: is C₁-C₂₀alkyl; hydroxy substituted C₁-C₂₀alkyl; phenyl; phenyl-C₁-C₃alkyl; or C₁-C₆alkylcarbonyl; and most preferably compounds of formula (1c), wherein
- R₁, R₂ and R₃: are hydrogen; or C₁-C₂₀alkyl, or compounds of formula (1 c), wherein
- R₁, R₂ and R₃: are hydrogen; and
- R₄: is C₁-C₂₀alkyl; phenyl-C₁-C₅alkyl; or C₁-C₆alkylcarbonyl.

Mostly preferred are compounds of formula (1c), wherein
- R₁,R₂ and R₃: are hydrogen; or C₁-C₂₀alkyl; or compounds of formula (1c), wherein
- R₁, R₂ and R₃: are hydrogen; and
- R₄: is C₁-C₂₀alkyl; phenyl-C₁-C₅alkyl; or C₁-C₆alkylcarbonyl.

In a further preferred embodiment of the present invention the personal care product contains an antimicrobial of formula 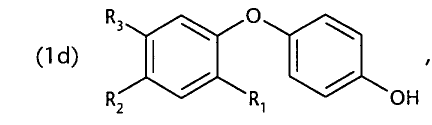 wherein
- R₁,R₂ and R₃: independently from each other are hydrogen; C₁-C₂₀alkyl; hydroxy-substituted C₁-C₂₀akyl; cyclo-C₅-C₇alkyl; phenyl-C₁-C₃alkyl, wherein at least one of the substituents R₁, R₂ or R₃ is not hydrogen.

Most preferred are compounds of formula (1d), wherein
- R₁ and R₃: independently of each other are C₁-C₂₀alkyl; and
- R₂: is hydrogen.

In a further preferred embodiment of the present invention the personal care product contains an antimicrobial of formula 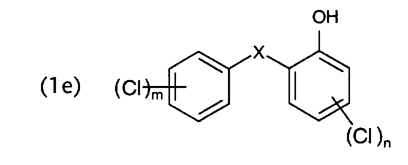 wherein
- X: is oxygen, or -CH₂-,
- m: 1 to 3, and
- n: is 1 or 2; and most preferably compounds of formula

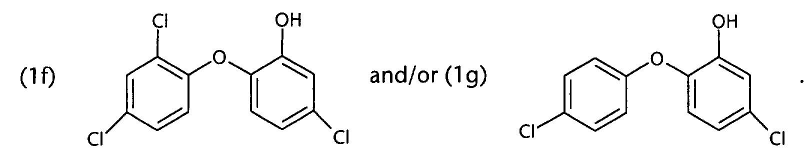

Preferred personal care product of the present invention comprise as Uv absorber a compound of formula (2), wherein
R₆ and R₇ independently from each other are C₁-C₈-alkyl; and/or
a compound of formula (3), in which R₈ and R₉, independently, are C₁-C₄alkyl or C₁-C₄alkoxy; and/or
a compound of formula (4a) or a mixture of compounds of formula 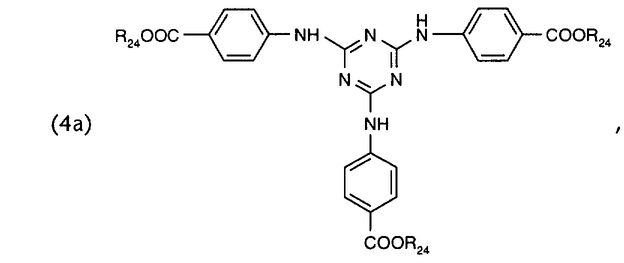 in which
the individual radicals R₂₄ are the same or different and each is hydrogen; an alkali metal; an ammonium group N(R₂₅)₄, in which R₂₅ is hydrogen or an organic radical; C₁-C₂₀alkyl; or a polyoxyethylene radical which contains from 1 to 10 ethylene oxide units and the terminal OH group of which may be etherified by a C₁-C₃alcohol; and/or
a compound of formula (5), in which
- R₁₄: is C₁-C₄alkyl or hydrogen; and
- R₁₅: is C₁₋₂alkyl or α,α-dimethylbenzyl; and/or
a compound of formula (6), in which
- R₁₅: is C₁₋₂alkyl or α,α-dimethylbenzyl; and/or
a compound of formula (7), in which
- R₁₆: is C₁-C₅alkyl or phenyl optionally substituted by one, two or three substituents selected from OH, C₁-C₄alkyl, C₁-C₄alkoxy or CO-OR₁₂ in which R₁₂ has its previous significance;
- R₁₇, R₁₈, R₁₉ and R₂₀: are the same or different and each is C₁-C₅alkyl or hydrogen;
- Y: is N or O; and
- m: has its previous significance; and/or
a compound of formula (8), in which
- R₂₁: is hydroxy; methoxy; or ethoxy,
- R₂₂: is hydrogen; or C₁-C₂alkyl; and
- R₂₃: is phenyl; 4-methoxyphenyl; or the phenylaminocarbonyl group; and/or
a compound of formula (9), in which
- M: is hydrogen, sodium, magnesium, calcium or zinc.

Further hydroxydiphenylether compounds, which can be used in the present invention, are listed in the following Table 1:

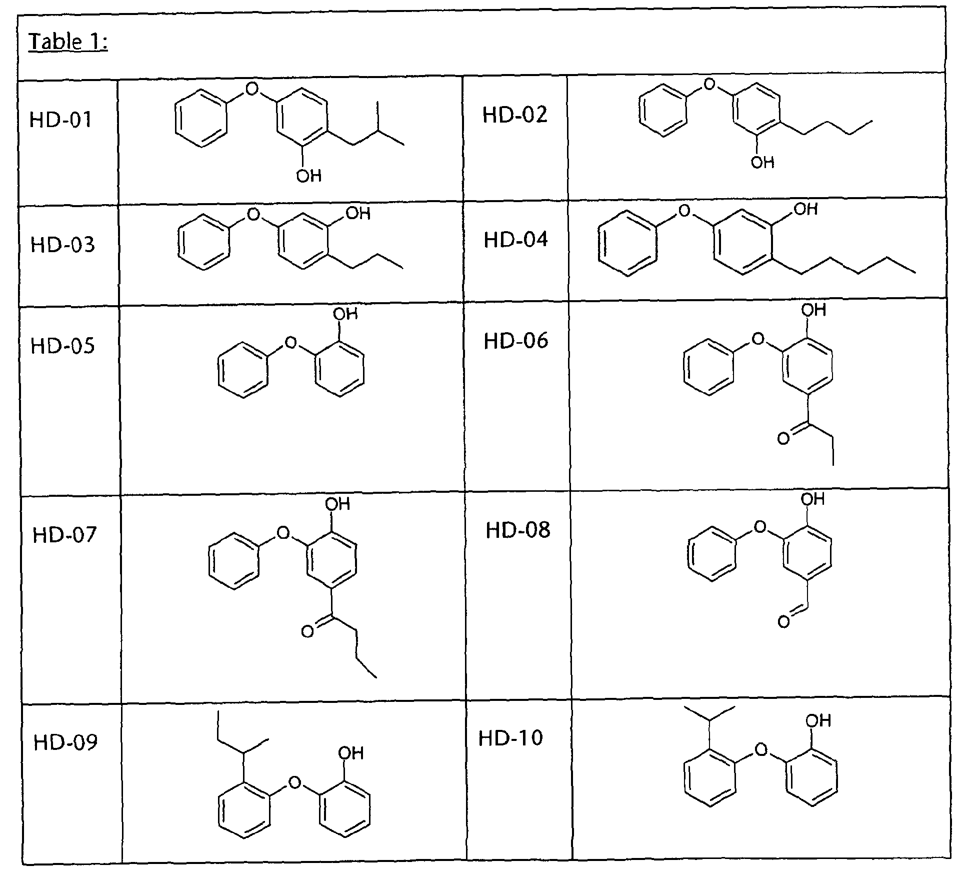

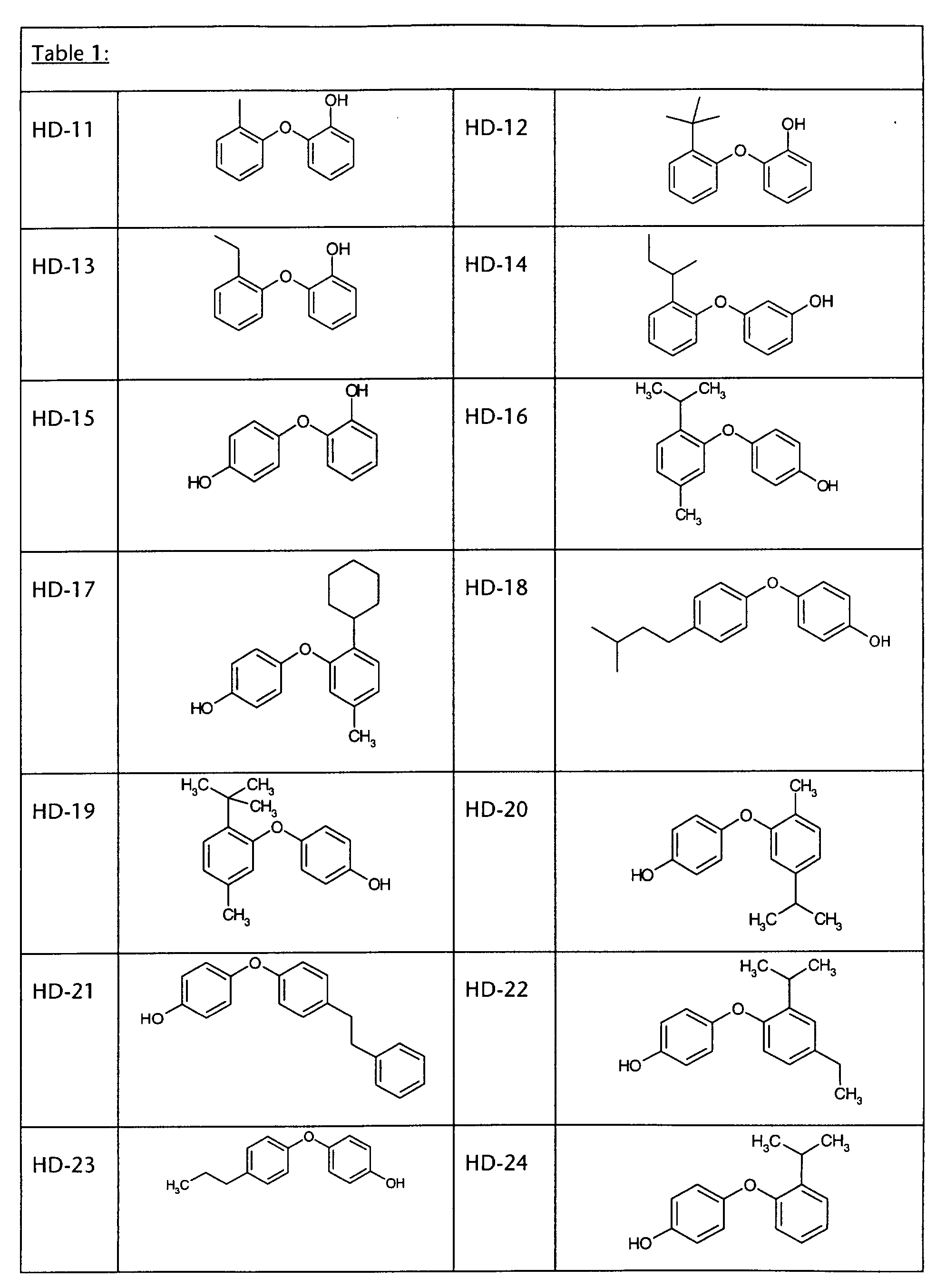

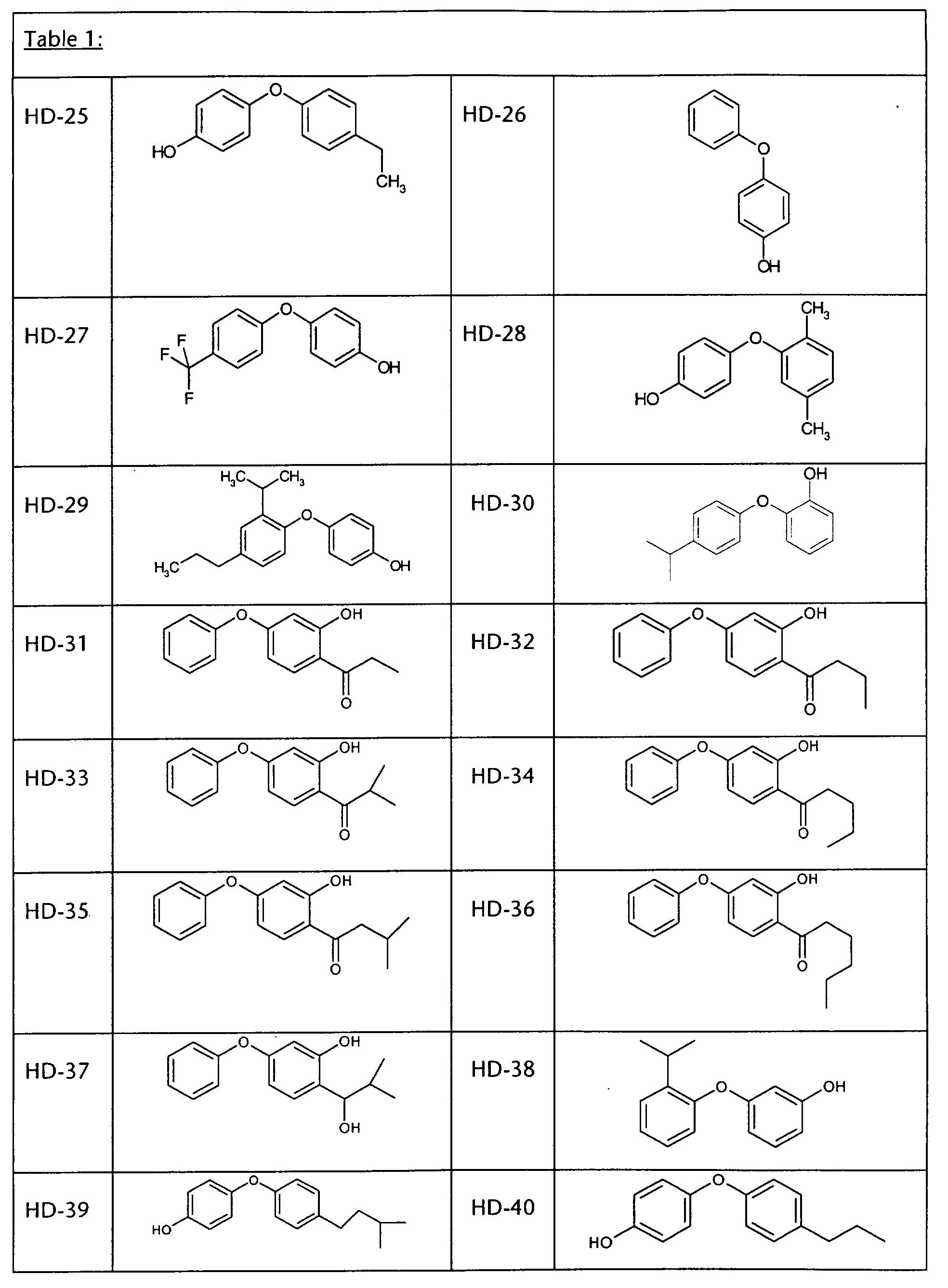

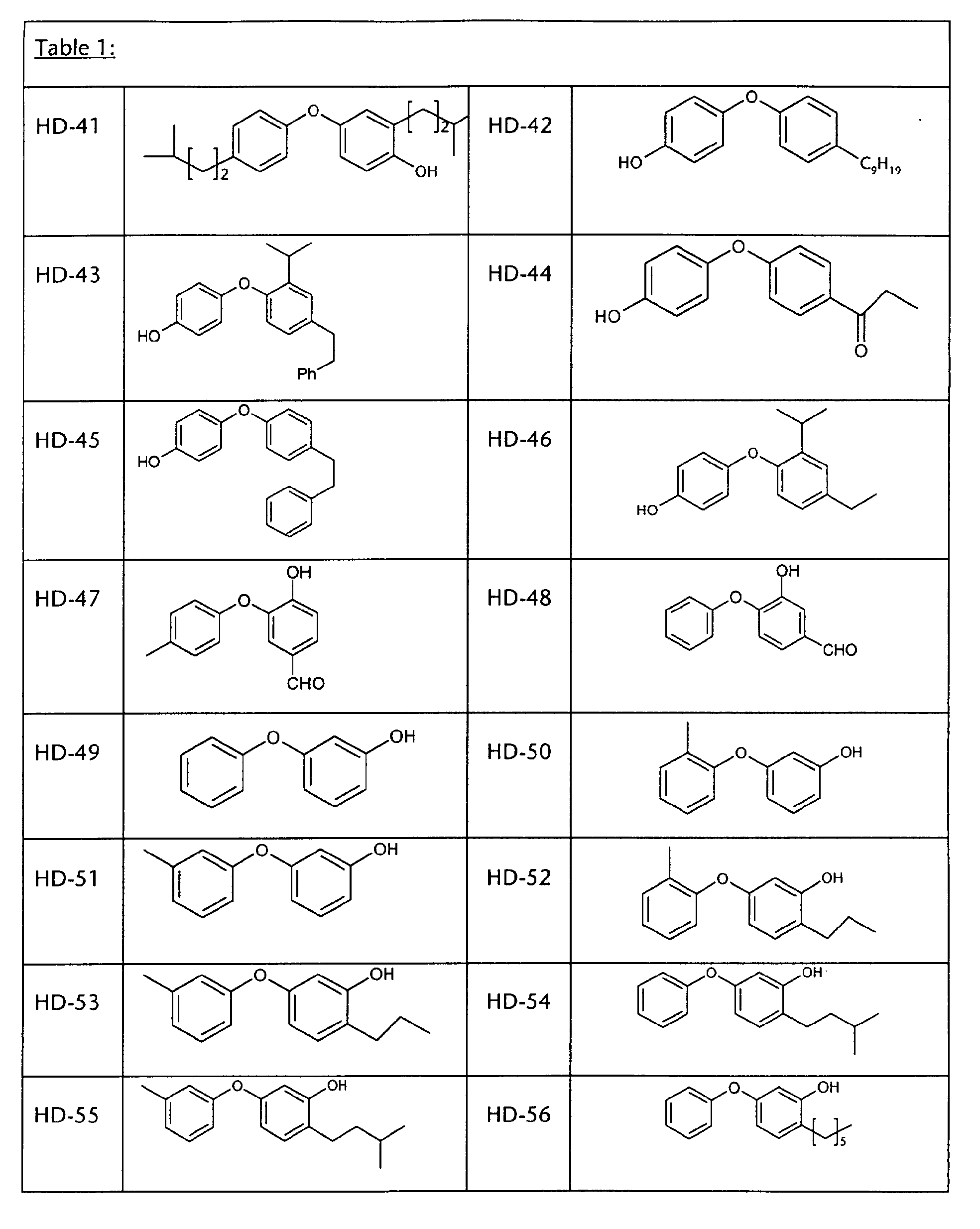

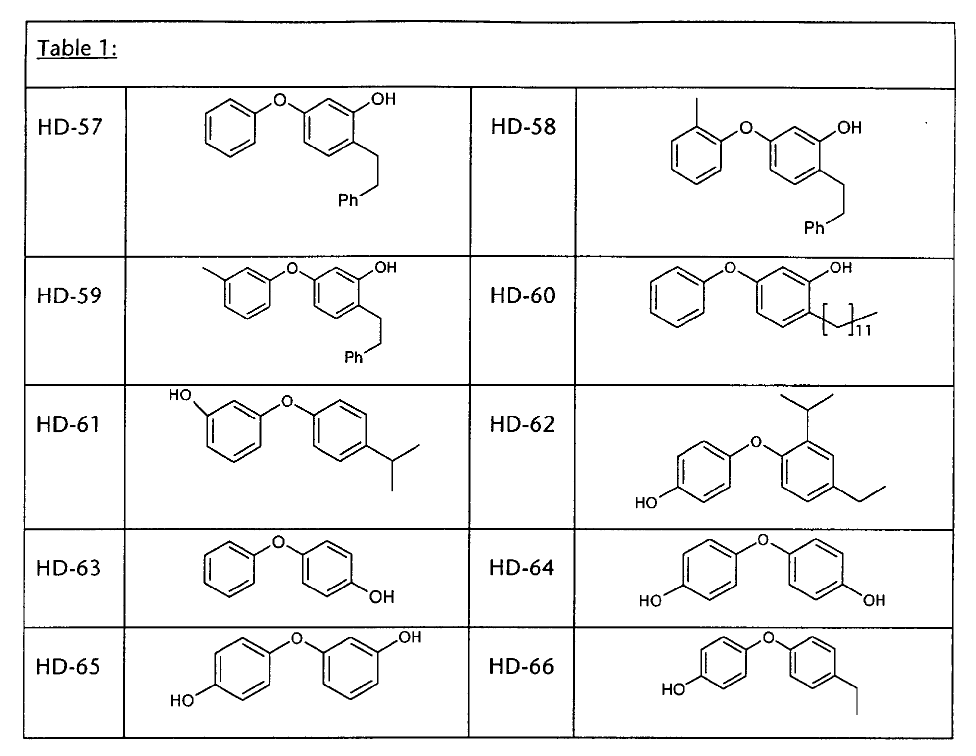

The method for producing compounds of formula (1 - (9) is for example disclosed in WO 0222941, EP-A2-1053989 and US 5980872.

In a further embodiment of the present invention the personal care product comprises as a further component (c)at least one antioxidant of the following formulae 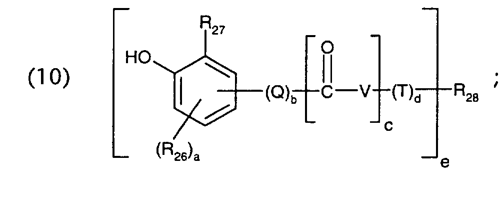 and/or 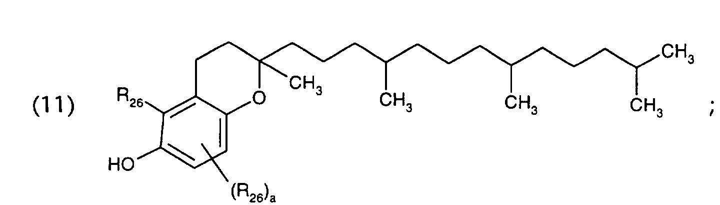 and/or 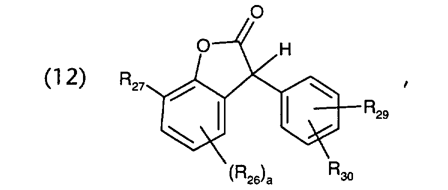 wherein in formulae (10), (11) and (12)
- R₂₆: is hydrogen; C₁-C₂₂alkyl; C₁-C₂₂alkylthio; C₅-C₇cycloalkyl; phenyl; C₇-C₉phenylalkyl or SO₃M,
- R₂₇: is C₁-C₂₂alkyl; C₅-C₇cycloalkyl; phenyl or C₇-C₉phenylalkyl,
- Q: is -CₘH₂ₘ-; 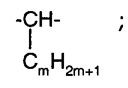 -CₘH₂ₘ-NH; a radical of formula 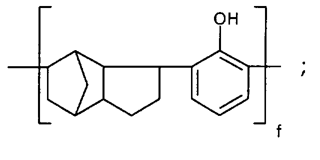 or 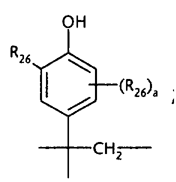
- T: is -CₙH₂ₙ-; -(CH₂)ₙ-O-CH₂-; 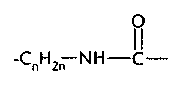 or a radical of formula (1c) 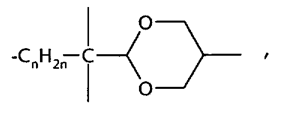
- V: is -O- or -NH-,
- a: is 0; 1; or 2,
- b, c and d: are each independently of one another 0 or 1,
- e: is an integer from 1 to 4,
- f: is an integer from 1 to 3, and
- m, n and p: are each independently of one another an integer from 1 to 3,
if e = 1, then
- R₂₈: is hydrogen; M; C₁-C₂₂alkyl; C₅-C₇cycloalkyl; C₁-C₂₂alkylthio; C₂-C₁₈alkenyl; C₁-C₁₈-phenylalkyl; a radical of formula 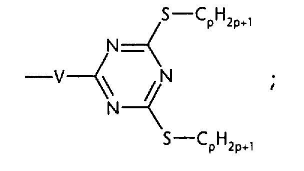 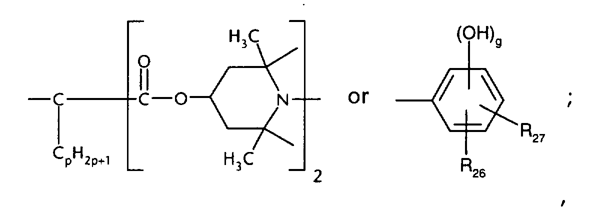
- M: is alkali; ammonium,
if e = 2, then
- R₂₈: is a direct bond; -CH₂-; 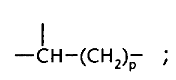
-O- or -S-, if
e = 3, then
- R₂₈: is the radical of formula 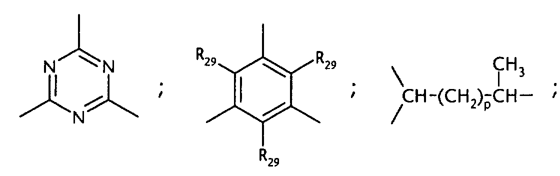
or 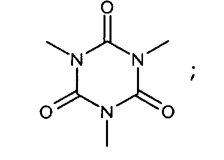 If e = 4, then
- R₂₈: is 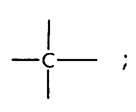 or a direct bond and
- R₂₉ and R₃₀: are each independently of the other hydrogen; or C₁-C₂₂alkyl.

Preferred antioxidants can be found in Table 1 of EP 1135093 and of EP 1126811. The process for production of these antioxidants can also be found in these two patent applications.

Personal care products are shampoos, bath- and shower additives, hair-care products, liquid and bar soaps, lotions and crèmes, deodorants, other aqueous or alcoholic solutions, for example cleaning solutions for the skin, moist cleaning sheets, oils and powders.

Preferred personal care products are deodorants and bar soaps.

The personal care composition according to the present invention comprises

| | |
|---|---|
| 0.01 to 15 | wt-% (wt-%) of component (a); |
| 0.05 to 15 | wt-% of component (b); and |
| 0 to 15 | wt-% of component (c); |

and cosmetically tolerable carriers or auxiliaries.

The above percentages are in each case percentages by weight, based on the total weight of the composition.

Preferred is a personal care composition according to the present invention comprises
(a) 0.01 to 15, more preferably 0.1 to 10 weight-% (wt-%) of at least one compound of formula (HD-01) to (HD-66) of Table 1;
(b) 0.05 to 15 wt-%, more preferably 0.1 to 10 wt-% of a compound or a mixture of such compounds, and
(c) 0 to 15 wt-% of a compound of formula (10);
and cosmetically tolerable carriers or auxiliaries.

The personal care composition according to the invention can be formulated as a water-in-oil or oil-in-water emulsion, as an oil-in-alcohol lotion, as a vesicular dispersion of an ionic or non-ionic amphiphilic lipid, as a gel, solid stick, aerosol formulation or a surfactant based formulation, such as a soap or skin cleanser.

As a water-in-oil or oil-in-water emulsion, the cosmetically compatible auxiliary preferably contains 5 to 50% of an oil phase, 5 to 20% of an emulsifier and 30 to 90% of water. The oil phase can in this case contain any oil suitable for cosmetic formulations, e.g. one or more hydrocarbon oils, a wax, a natural oil, a silicone oil, a fatty acid ester or a fatty alcohol. Preferred mono- or polyols are ethanol, isopropanol, propylene glycol, hexylene glycol, glycerol and sorbitol.

Any conventionally usable emulsifier can be used for the cosmetic composition according to the invention, for example one or more ethoxylated esters of natural derivatives, e.g. polyethoxylated esters of hydrogenated castor oil; or a silicone oil emulsifier, e.g. a silicone polyol; an optionally ethoxylated fatty acid soap; an ethoxylated fatty alcohol; an optionally ethoxylated sorbitan ester; an ethoxylated fatty acid; or an ethoxylated glyceride.

The cosmetic composition may also comprise further components, e.g. emollients, emulsion stabilisers, skin humectants, skin tanning accelerators, thickeners, such as xanthan, moisture-retention agents, such as glycerol, preservatives, perfumes and colourings.

The preparation of the cosmetic composition can be effected by physically mixing the anti-microbial(s) with the auxiliary by customary methods, for example by simply stirring the individual components together.

Cosmetic formulations include a very wide range of cosmetic products. Suitable products are, for example, especially the following:
- skin-care products, for example skin washing and cleansing products in the form of bars of soap or liquid soaps, syndets or washing pastes,
- bath products, for example liquid (foam baths, milks, shower products) or solid bath products, such as bath pearls and bath salts;
- skin-care products, such as skin emulsions, multiple emulsions or skin oils;
- decorative body-care products, for example face make-ups in the form of day or powder creams, face powders (lose and compressed), rouge or cream make-ups, eye-care products, for example eye shadow products, mascara, eyeliners, eye creams or eye-fix creams; lip-care products, for example lipstick, lip gloss, lip liner, nail-care products, such as nail varnish, nail varnish remover, nail hardeners or cuticle removers;
- feminine hygiene products, such as feminine hygiene washing lotions or sprays;
- foot-care products, for example foot baths, foot powders, food creams or foot balms, special deodorants and antiperspirants or products for scrubbing off callouses;
- sunscreens, such as sun milks, lotions, creams, oils, sunblockers or tropicals, pre-sun products or after-sun products;
- suntanning products, for example self-tanning creams;
- depigmenting products, for example products for bleaching or lightening skin;
- insect repellents, for example insect oils, lotions, sprays or sticks;
- deodorants, for example deodorant sprays, non-aerosol sprays, deodorant gels, sticks or roll-ons;
- antiperspirants, for example antiperspirant sticks, creams or roll-ons;
- products for cleansing and treating impure skin, for example syndets (solid or liquid), peeling or scrubbing products or peeling masks;
- chemical depilatory products, for example depilatory powders, liquid depilatory products, creamy or pasty depilatory products, depilatory gels or aerosol foams;
- shaving products, for example shaving soap, foaming shaving creams, non-foaming shaving creams, shaving foams and gels, preshaving products for dry shaving, aftershaves or aftershave lotions;
- scents, for example perfumes (Eau de Cologne, Eau de Toilette, Eau de Parfum, Parfum de Toilette, perfume), perfume oils or perfume creams;
- products for oral and dental hygiene as well as for dentures, for example toothpastes, tooth gels, tooth powders, mouth-wash concentrates, anti-plaque mouth-washes, denture cleaning products or denture adhesion products;
- cosmetic formulations for hair treatment, for example hair washes in the form of shampoos, hair conditioners, hair-care products, for example pretreatment products, hair tonics, hair styling creams and gels, pomades, hair rinses, deep conditioning treatments, intensive hair care treatments, hair setting products, for example waving agents for perms (hot wave, mild wave, cold wave), hair straightening products, liquid hair fixatives, hair foams, hair sprays, bleaching agents, for example hydrogen peroxide solutions, bleaching shampoos, bleaching creams, bleaching powders, bleaching pastes or oils, temporary, semitemporary or permanent hair dyes, products containing self-oxidising dyes, or natural hair dyes, such as henna or camomile.

The antioxidant(s) are optionally present in the novel body-care product in a concentration of 50 to 1000 ppm.

An improved antimicrobial soap has, for example, the following composition:

| | |
|---|---|
| 0.01 to 5 wt-% | of at least one compound of the formula (1) |
| 0.2 to 1 % wt-% | of a compound of formula (2) - (9) or a mixture thereof |
| 1 to 10 wt-% | of stearic acid |
| to 100 wt% | of soap base, for example the sodium salts of tallow fatty and coconut fatty acid or glycerols. |

A preferred improved antimicrobial soap has, for example, the following composition:

| | |
|---|---|
| 0.01 to 5 wt-% | of at least one compound of the formula (HD-01) to (HD-66) of Table 1 |
| 0.2 to 1 % wt-% | of at least compound of formula (2) |
| 1 to 10 wt-% | of stearic acid |
| to 100 wt% | of soap base, for example the sodium salts of tallow fatty and coconut fatty acid or glycerols. |

An improved shampoo has, for example, the following composition:
0.01 to 5 wt-% of at least one compound of the formula (1)
0.2 to 1 % wt-% of a compound of formula (2) - (9) or a mixture thereof
12.0 wt-% of sodium laureth-2-sulfate,
4.0 wt-% of cocamidopropylbetaine,
3.0 wt-% of NaCI and
water to 100 wt-%.

An improved deodorant has, for example, the following composition:
0.01 to 5 wt-% of at least one compound of the formula (1)
0.2 to 1 % wt-% of a compound of formula (2) to (9) or a mixture thereof
60 wt-% of ethanol,
0.3 wt-% of perfume oil and
water to 100%.

The personal care formulations listed above can be in a very wide range of forms of presentation, for example
- in the form of liquid formulations as an O/W emulsion,
- in the form of a gel,
- in the form of an oil, cream, milk or lotion,
- in the form of a powder, lacquer, pellets or make-up,
- in the form of a stick,
- in the form of a spray (spray with propellant or pumping spray) or an aerosol,
- in the form of a foam, or
- in the form of a paste.

The oral hygiene composition may comprise an additional antibacterial enhancing agent, for example an anionic polymeric polycarboxylate, a dehydrated polyphosphate salt, a compound which provides a source of fluoride ions, a polishing material, including siliceous material or sodium bicarbonate, an orally acceptable vehicle, including a water-phase with humectant, thickeners, surface-active agents and a flavoring or sweetening material.

The following examples illustrate the present invention. The percentages are weight percentages and the temperatures are given in degree Celsius.

### Example 1:

496.75 g of a soap (Mettler-Grundseife 400010, which is a product of Mettler AG, Hornussen, Switzerland), 0.25 g of ethylenediamine tetra-acetic acid (EDTA) and 1 g of 4-(2-t-butyl-5-methylphenoxy)-phenol are placed in a mixer (i.e. a Z-blade mixer).

50 ml of a 1:1 mixture of glycerine and water, which additionally contains 2 g of a mixture consisting of

| | |
|---|---|
| 50 wt-% | of methylene bis-benztriazolyl tetramethylbutylphenol, |
| 7.5 wt-% | of decyl glucosid |
| 0.2 wt-% | of xanthan gum |
| 0.4 wt-% | of propylenglycol and |
| 41.9 wt-% | of water |

is added. The mass is mixed for about 20 minutes. The mass is then transferred to a three-roll mill, plodded into a billet, cut and finally stamped into bars.

### Example 2 (comparison Example)

499.75 g of a soap (Mettler-Grundseife 400010, which is a product of Mettler AG, Hornussen, Switzerland) and 0.25 g of EDTA are placed in a mixer (i.e. a Z-blade mixer). 50 ml of a 1:1 mixture of glycerine and water is added. The mass is mixed for about 20 minutes. The mass is then transferred to a three-roll mill, plodded into a billet, cut and finally stamped into bars.

### Example 3 (comparison Example)

498.75 g of a soap (Mettler-Grundseife 400010, which is a product of Mettler AG, Hornussen, Switzerland), 0.25 g of EDTA and 1 g of 4-(2-t-butyl-5-methylphenoxy)-phenol are placed in a mixer (i.e. a Z-blade mixer). 50 ml of a 1:1 mixture of glycerine and water is added. The mass is mixed for about 20 minutes. The mass was then transferred to a three roll mill, plodded into a billet, cut and finally stamped into bars.

### Example 4 (comparison Example)

497.75 g of a soap (Mettler-Grundseife 400010, which is a product of Mettler AG, Hornussen, Switzerland), 0.25 g of EDTA, 1 g of 4-(2-t-butyl-5-methylphenoxy)-phenol and 1 g of TiO₂ P25 (a product of DEGUSSA) are placed in a mixer (i.e. a Z-blade mixer). 50 ml of a 1:1 mixture of glycerine and water is added. The mass is mixed for about 20 minutes. The mass is then transferred to a three roll mill, plodded into a billet, cut and finally stamped into bars.

The bar soaps are exposured to light in a Suntest XLS apparatus. The exposure time has been between 2 and 8 hours. The light intensity has been 765 W/m². The degree of whiteness (Method of Ganz) of the soap bars of each example are determined after the exposure. The whiteness values of the respective dried goods are measured with a DCl/SF 500 spectrophotometer according to the Ganz method.

The Method of Ganz is described in detail in the Ciba-Geigy Review, 1973/1, and also in the article "Whiteness Measurement", ISCC Conference on Fluorescence and the Colorimetry of Fluorescent Materials, Williamsburg, February 1972, published in the journal of Color and Appearance, 1, No.5 (1972).

The results are given in Table 2.

**Table 2**

| Example | Degree of Whiteness | | |
|---|---|---|---|
| | 2h | 4h | 8h |
| 1 | 59 | 69 | 79 |
| 2 | 64 | 79 | 94 |
| 3 | 28 | 37 | 42 |
| 4 | 48 | 17 | 2 |

The soap of example 1 shows an improved degree of whiteness in comparison to the soap with only the antimicrobial agent (example 3) or to the soap with TiO₂ (example 4). TiO₂ is known as a compound, which usually protects bar soaps against discolouring (yellowing).

## Claims

1. Personal care product with antimicrobial action, comprising
(a) as antimicrobial a compound of formula 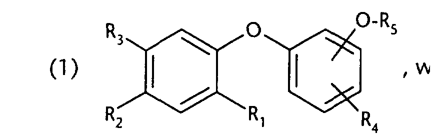 wherein
R₁, R₂ and R₃ independently from each other are hydrogen; hydroxy; halogen; C₁-C₂₀alkyl; hydroxy-substituted C₁C₂₀alkyl; C₅-C₇cycloalkyl; C₁-C₂₀alkoxy; C₁-C₆alkylcarbonyl; phenyl; or phenyl-C₁-C₃alkyl;
R₄ is hydrogen; halogen; C₁-C₂₀alkyl; hydroxy-substituted C₁-C₂₀alkyl; C₅-C₇cycloalkyl; hydroxy; formyl; acetonyl; allyl; carboxy; carboxy-C₁-C₃alkyl; carboxyallyl; C₂-C₂₀alkenyl; C₁-C₆-alkylcarbonyl; C₁-C₃alkylcarbonyl-C₁-C₃alkyl; phenyl; or phenyl-C₁-C₃alkyl; and
R₅ is hydrogen; C₁-C₂₀alkoxy; or C₁-C₆alkylcarbonyl; and
(b) a UV absorber selected from
(b₁) a compound of formula 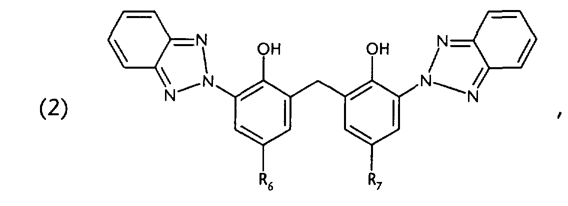 wherein
R₆ and R₇ independently from each other signify C₁-C₁₂-alkyl;
(b₂) a compound of formula 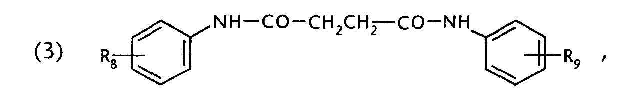 in which R₈ and R₉, independently, are C₁-C₁₈alkyl or C₁-C₁₈alkoxy;
(b₃) a compound of formula 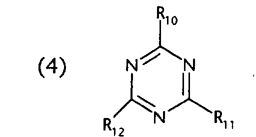 in which
R₁₀, R₁₁ and R₁₂, independently, are H; OH; C₁-C₁₈alkoxy; NH₂; NH-R₁₃ or N(R₁₃)₂in which R₁₃ is C₁-C₁₈alkyl; OR₁₃; phenyl, phenoxy or anilino, or pyrrolo, in which the respective phenyl, phenoxy or anilino, or pyrrolo moieties are optionally substituted by one, two or three substitutents selected from OH, carboxy, CO-NH₂, C₁-C₁₈alkyl or C₁-C₁₈alkoxy; C₁-C₁₈Carboxyalkyl; C₅-C₈cycloalkyl; a methylidenecamphor group; a group - (CH=CH)ₘC(=O)-OR₁₃ in which m is 0 or 1; or a group 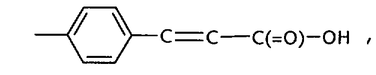
or the corresponding alkali metal, ammonium, mono-, di- or tri-C₁-C₄alkylammonium, mono-, di- or tri-C₂-C₄alkanolammonium salts, or the C₁-C₁₈alkyl esters thereof;
(b₄) a compound of formula 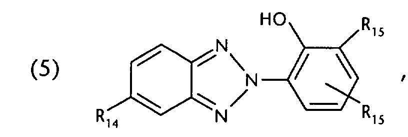 in which
R₁₄ is C₁-C₁₈alkyl or hydrogen; and
R₁₅ is C₁-C₁₈alkyl, optionally substituted by phenyl,
(b₅) a compound of formula 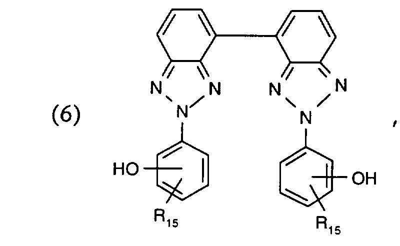 in which R₁₅ has its previous significance;
(b₆) a compound of formula
(7) R₁₆-(Y)ₘ-CO-C(R₁₇)=C(R₁₈)-N(R₁₉)(R₂₀),
in which
R₁₆ is C₁-C₁₈alkyl or phenyl optionally substituted by one, two or three substituents selected from OH, C₁-C₁₈alkyl, C₁-C₁₈alkoxy or CO-OR₁₃;
R₁₇, R₁₈, R₁₉ and R₂₀ are the same or different and each is C₁-C₁₈alkyl; or hydrogen;
Y is N; or O; and
m has its previous significance;
(b₇) a compound of formula 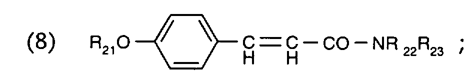 n which
R₂₁ is hydroxy; or C₁-C₄alkoxy,
R₂₂ is hydrogen; or C₁-C₄alkyl; and
R₂₃ is -(CONH)ₘ-phenyl in which m has its previous significance and the phenyl group is optionally substituted by one, two or three substituents selected from OH, C₁-C₁₈alkyl, C₁-C₁₈alkoxy or CO-OR₁₃; and
(b₈) a compound of formula 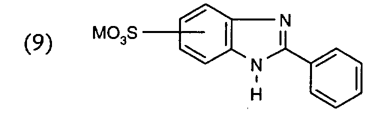 in which
M is hydrogen; an alkali metal; an alkaline earth metal; or zinc.

2. A personal care product according to claim 1 comprising as antimicrobial (a) a compound of formula (1), wherein
R₁ is C₁-C₁₆alkyl; and
R₂, R₃ R₄ and R₅ are hydrogen.

3. A personal care product according to claim 1 comprising as antimicrobial (a) a compound of formula 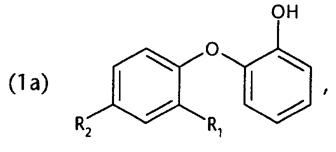 wherein
R₁ and R₂ independently of each other are hydrogen; C₁-C₂₀alkyl; or hydroxy-substituted C₁-C₂₀alkyl; wherein at least one of the substituents R₁ and R₂ is not hydrogen.

4. A personal care product according to claim 1 comprising as antimicrobial (a) a compound of formula 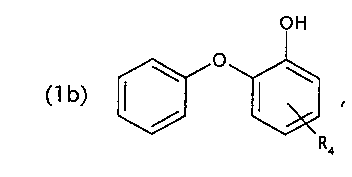 wherein
R₄ is carboxy; carboxy-C₁-C₃alkyl; C₁-C₆alkylcarbonyl; or C₁-C₃alkylcarbonyl-C₁-C₃alkyl.

5. A personal care product according to Claim 1 comprising as antimicrobial (a) a compound of formula 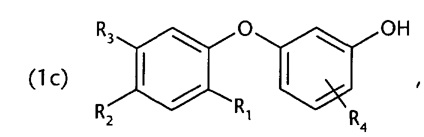 wherein
R₁, R₂ and R₃ independently from each other are hydrogen; hydroxy; C₁-C₂₀alkyl; or hydroxy-substituted C₁C₂₀alkyl; and
R₄ is C₁-C₂₀alkyl; hydroxy substituted C₁-C₂₀alkyl; phenyl; phenyl-C₁-C₃alkyl; or C₁-C₆alkylcarbonyl.

6. A personal care product according to claim 5, wherein
R₁, R₂ and R₃ are hydrogen; or C₁-C₂₀alkyl.

7. A personal care product according to claim 5 or 6, wherein
R₁, R₂ and R₃ are hydrogen; and
R₄ is C₁-C₂₀alkyl; phenyl-C₁ -C₅alkyl; or C₁-C₆alkylcarbonyl.

8. A personal care product according to claim 1 comprising as antimicrobial (a) a compound of formula 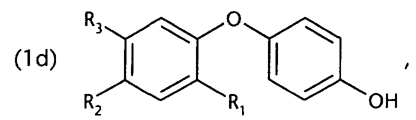 wherein
R₁, R₂ and R₃ independently from each other are hydrogen; C₁-C₂₀alkyl; hydroxy-substituted C₁-C₂₀akyl; cyclo-C₅-C₇alkyl; phenyl-C₁-C₃alkyl, wherein at least one of the substituents R₁, R₂ or R₃ is not hydrogen.

9. A personal care product according to claim 8, wherein
R₁ and R₃ independently of each other are C₁-C₂₀alkyl; and
R₂ is hydrogen.

10. A personal care product according to claim 1, comprising as antimicrobial (a) a compound of formula 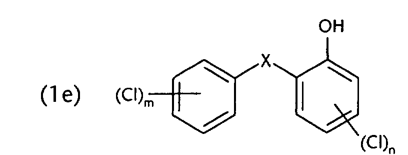 wherein
X is oxygen, or -CH₂-;
m 1 to 3; and
n is 1 or 2.

11. A personal care product according to claim 1 or 10 comprising as antimicrobial (a) a compound of formula

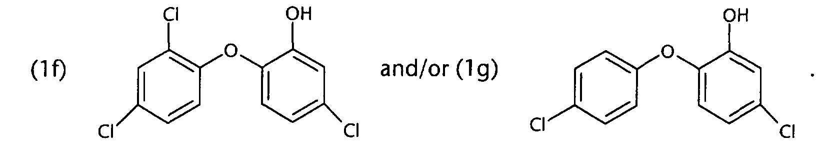

12. A personal care product according to anyone of claim 1 to 11 comprising as UV absorber (b) a compound of formula (2), wherein
R₆ and R₇ independently from each other are C₁-C₈-alkyl,
and/or
a compound of formula (3), in which R₈ and R₉, independently, are C₁-C₄alkyl or C₁-C₄alkoxy,
and/or
a compound of formula (4a) or a mixture of compounds of formula 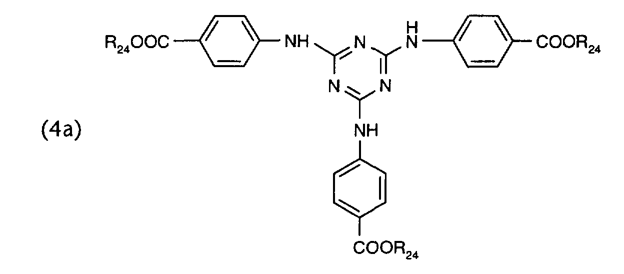 in which
the individual radicals R₂₄ are the same or different and each is hydrogen; an alkali metal; an ammonium group N(R₂₅)₄ in which R₂₅ is hydrogen or an organic radical; C₁-C₂₀alkyl; or a polyoxyethylene radical which contains from 1 to 10 ethylene oxide units and the terminal OH group of which may be etherified by a C₁-C₃alcohol,
and/or
a compound of formula (5), in which
R₁₄ is C₁-C₄alkyl or hydrogen; and
R₁₅ is C₁₋₂alkyl or α,α-dimethylbenzyl,
and/or
a compound of formula (6), in which
R₁₅ is C₁₋₂alkyl or α,α-dimethylbenzyl,
and/or
a compound of formula (7), in which
R₁₆ is C₁-C₅alkyl or phenyl optionally substituted by one, two or three substituents selected from OH, C₁-C₄alkyl, C₁-C₄alkoxy or CO-OR₁₂ in which R₁₂ has its previous significance;
R₁₇, R₁₈, R₁₉ and R₂₀ are the same or different and each is C₁-C₅alkyl or hydrogen,
Y is N or O; and
m has its previous significance,
and/or
a compound of formula (8), in which
R₂₁ is hydroxy; methoxy or ethoxy,
R₂₂ is hydrogen or C₁-C₂alkyl; and
R₂₃ is phenyl, 4-methoxyphenyl or the phenylaminocarbonyl group,
and/or
a compound of formula (9), in which
M is hydrogen, sodium, magnesium, calcium or zinc.

13. A personal care product according to anyone of Claim 1 to 12 comprising as a further component (c) at least one antioxidant of the following formulae 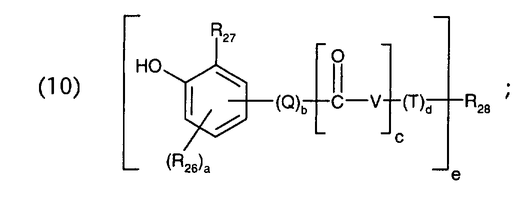 and/or 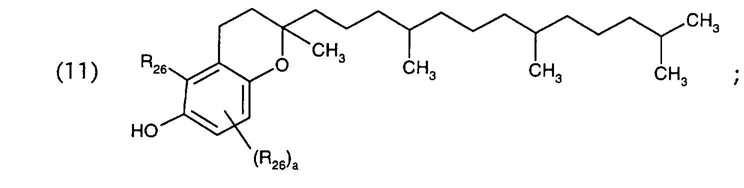 and/or 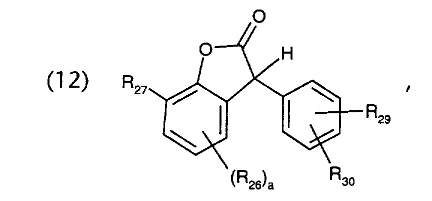 wherein in formulae (10), (11) and (12)
R₂₆ is hydrogen; C₁-C₂₂alkyl; C₁-C₂₂alkylthio; C₅-C₇cycloalkyl; phenyl; C₇-C₉phenylalkyl or SO₃M,
R₂₇ is C₁-C₂₂alkyl; C₅-C₇cycloalkyl; phenyl or C₇-C₉phenylalkyl,
Q is -CₘH₂ₘ-; 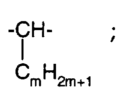 -CₘH₂ₘ-NH; a radical of formula 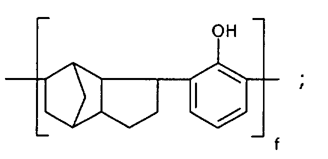 or 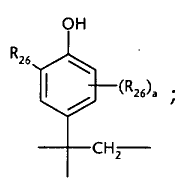
T is -CₙH₂ₙ-; -(CH₂)ₙ-O-CH₂-; 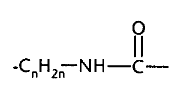 or a radical of formula (1c) 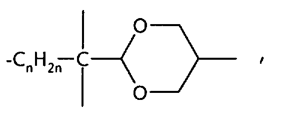
V is -O- or -NH-,
a is 0; 1; or 2,
b, c and d are each independently of one another 0 or 1,
e is an integer from 1 to 4,
f is an integer from 1 to 3, and
m, n and p are each independently of one another an integer from 1 to 3,
if e = 1, then
R₂₈ is hydrogen; M; C₁-C₂₂alkyl; C₅-C₇cycloalkyl; C₁-C₂₂alkylthio; C₂-C₁₈alkenyl; C₁-C₁₈-phenylalkyl; a radical of formula 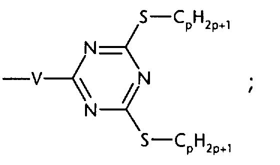 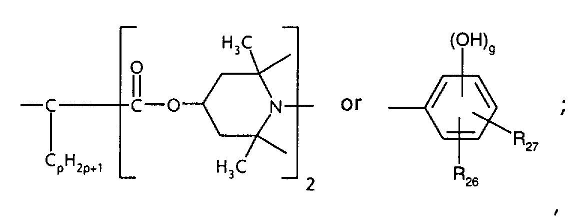
M is alkali; ammonium,
if e = 2, then
R₂₈ is a direct bond; -CH₂-; 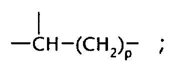
-O- or -S-, if e = 3, then
R₂₈ is the radical of formula 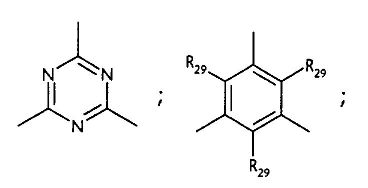 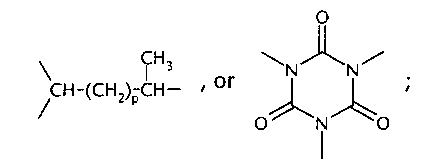
if e = 4, then
R₂₈ is 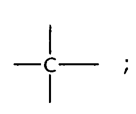
or a direct bond and
R₂₉ and R₃₀ are each independently of the other hydrogen; or C₁-C₂₂alkyl.

14. A personal care product according to claim 1 comprising
(a) as antimicrobial a compound of formula 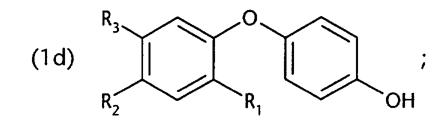
(b) as UV absorber a compound of formula 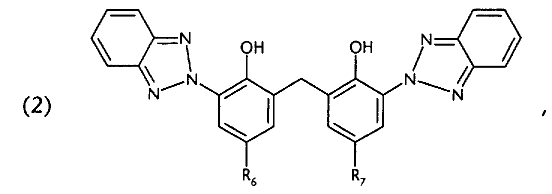
wherein
R₁, R₂ and R₃ independently from each other are hydrogen; C₁-C₂₀alkyl; hydroxy-substituted C₁-C₂₀akyl; cyclo-C₅-C₇alkyl; phenyl-C₁-C₃alkyl, wherein at least one of the substituents R₁, R₂ or R₃ is not hydrogen; and
R₆ and R₇ independently from each other are C₁-C₁₂alkyl.

15. A personal care product according to anyone of Claim 1 to 14, wherein the personal care product is a shampoo, a bath- and shower additive, a hair-care product, a liquid soap, a bar soap, a lotion, a crème, a deodorant, a solution for the skin, a moist cleaning sheet, an oil and a powder.

16. A personal care product according to anyone of Claim 1 to 14, wherein the personal care product is a deodorant or a bar soap.

17. A personal care product according to anyone of Claim 1 to 16 comprising
0.01 to 15 wt-% of component (a);
0.05 to 15 wt-% of component (b); and
0 to 15 wt-% of component (c);
and cosmetically tolerable carriers or auxiliaries.
